(19) Europäisches Patentamt
European Patent Office
Office européen des brevets

(11) Veröffentlichungsnummer: **0 342 474 A2**

(12) **EUROPÄISCHE PATENTANMELDUNG**

(21) Anmeldenummer: **89108284.4**

(51) Int. Cl.4: **A61K 31/55**

(22) Anmeldetag: **09.05.89**

(30) Priorität: **17.05.88 DE 3816709**

(43) Veröffentlichungstag der Anmeldung: **23.11.89 Patentblatt 89/47**

(84) Benannte Vertragsstaaten: **AT BE CH DE FR GB IT LI LU NL SE**

(71) Anmelder: **Dr. Karl Thomae GmbH**
**Postfach 1755**
**D-7950 Biberach 1(DE)**

(72) Erfinder: **Eberlein, Wolfgang, Dr. Dipl.-Chem.**
**Obere Au 6**
**D-7950 Biberach 1(DE)**
Erfinder: **Trach, Volker, Dr.**
**Probststrasse 7**
**D-7950 Biberach 1(DE)**
Erfinder: **Engel, Wolfhard, Dr. Dipl.-Chem**
**Mozartstrasse 13**
**D-7950 Biberach 1(DE)**
Erfinder: **Mihm, Gerhard, Dr. Dipl.-Chem**
**Nickeleshalde 5/1**
**D-7950 Biberach 1(DE)**
Erfinder: **Mayer, Norbert, Dr.**
**Friedrich-Ebert-Strasse 66**
**D-7950 Biberach 1(DE)**
Erfinder: **Doods, Henri, Dr.**
**Hornsteinweg 7**
**D-7951 Warthausen(DE)**
Erfinder: **Reichl, Richard, Dr.**
**Im Hippel 55**
**D-6535 Gau-Algesheim(DE)**

(54) **Verwendung von Diazepinonen zur Therapie von Störungen der Mikrozirkulation.**

(57) Für Diazepinone der allgemeinen Formel I

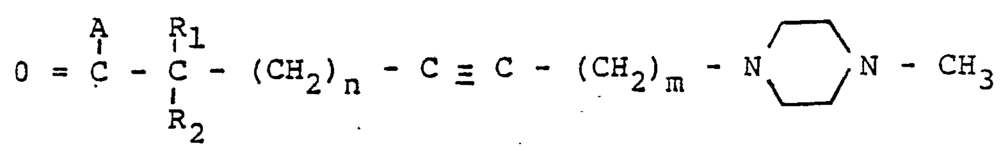
, (I)

in der
A die in 11-Stellung gebundene 5,11-Dihydro-6H-pyrido[2,3-b][1,4]benzodiazepin-6-on-, die in 5-Stellung gebundene 5,10-Dihydro-11H-dibenzo[b,e][1,4]diazepin-11-on-, die in 5-Stellung gebundene 5,11-Dihydro-10H-pyrido[3,2-b][1,4]benzodiazepin-10-on- oder die in 4-Stellung gebundene 4,9-Dihydro-10H-thieno[3,4-b][1,5]benzodiazepin-10-on-Gruppe, die in 1- und/oder in 3-Stellung durch Alkylreste mit 1 bis 4 Kohlenstoffatomen oder in 3-Stellung durch Chlor, Fluor oder Brom substituiert sein kann, die in 11-Stellung gebundene 6,11-Dihydro-5H-pyrido[2,3-b][1,5]benzodiazepin-5-on- oder die in 4-Stellung gebundene 1-Methyl-1,4,9,10-tetrahydropyrrolo[3,2-b][1,5]benzodiazepin-10-on-Gruppe, die in 3-Stellung durch Methyl oder Chlor substituiert sein kann,
$R_1$ ein Wasserstoffatom oder eine Alkylgruppe mit 1 bis 3 Kohlenstoffatomen,
$R_2$ eine Alkylgruppe mit 1 bis 3 Kohlenstoffatomen, und
n und m unabhängig voneinander die ganzen Zahlen 1 bis 3 bedeuten, gegebenenfalls ihre Isomeren bzw. Enantiomeren und ihre physiologisch verträglichen Säureadditionssalze wurde eine neue Anwendung als Mittel zur Therapie von Störungen der Mikrozirkulation, inbesondere aber zur Therapie von Schockzuständen, wie hämorrhagischer Schock, gefunden.

EP 0 342 474 A2

## Verwendung von Diazepinonen zur Therapie von Störungen der Mikrozirkulation

Die Erfindung betrifft die Verwendung von Diazepinonen der allgemeinen Formel I

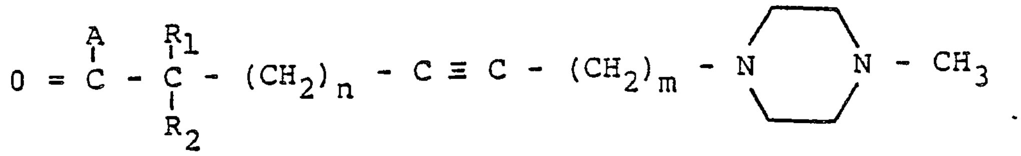

, (I)

in der
A die in 11-Stellung gebundene 5,11-Dihydro-6H-pyrido[2,3-b]-[1,4]benzodiazepin-6-on-, die in 5-Stellung gebundene 5,10-Dihydro-11H-dibenzo[b,e][1,4]diazepin-11-on-, die in 5-Stellung gebundene 5,11-Dihydro-10H-pyrido[3,2-b][1,4]benzodiazepin-10-on-, die in 4-Stellung gebundene 4,9-Dihydro-10H-thieno[3,4-b][l,5]-benzodiazepin-10-on-Gruppe, die in 1- und/oder in 3-Stellung durch Alkylreste mit 1 bis 4 Kohlenstoffatomen oder in 3-Stellung durch Chlor, Fluor oder Brom substituiert sein kann, die in 11-Stellung gebundene 6,11-Dihydro-5H-pyrido[2,3-b][1,5]benzodiazepin-5-on- oder die in 4-Stellung gebundene 1-Methyl-1,4,9,10-tetrahydropyrrolo[3,2-b][1,5]benzodiazepin-10-on-Gruppe, die in 3-Stellung durch Methyl oder Chlor substituiert sein kann,
$R_1$ ein Wasserstoffatom oder eine Alkylgruppe mit 1 bis 3 Kohlenstoffatomen,
$R_2$ eine Alkylgruppe mit 1 bis 3 Kohlenstoffatomen, und
n und m unabhängig voneinander die ganzen Zahlen 1 bis 3 bedeuten und von deren Säureadditionssalzen zur Therapie von Störungen der Mikrozirkulation, beispielsweise zur Therapie des hämorrhagischen Schocks und peripherer Durchblutungsstörungen unterschiedlicher Genese.

Aus EP-A-0 039 519 und 0 057 428 sowie aus US-A 3.660.380; 3.691.159; 4.213.984; 4.213.985; 4.210.648; 4.410.527; 4.424.225; 4.424.222 und 4.424.226 sind bereits kondensierte Diazepinone mit ulkushemmenden und magensaftsekretionshemmenden Eigenschaften bekannt.

Es ist aus der DE-3 611 097-A1 bekannt, daß die unter die obige allgemeine Formel I fallende Verbindung 5,11-Dihydro-11-[1-oxo-2-methyl-6-(4-methyl-1-piperazinyl)-4-hexinyl]-6H-pyrido[2,3-b][1,4]-benzodiazepin-6-on und deren Säureadditionssalze eine antithrombotische Wirkung besitzen. Die Verbindung, ihre Herstellung und Verarbeitung zu pharmazeutischen Anwendungsformen ist in dieser Offenlegungsschrift beschrieben. Die übrigen Verbindungen der obigen allgemeinen Formel I lassen sich in Analogie zu der in der DE-3 523 002-A1 beschriebenen Weise herstellen.

Es wurde nun überraschenderweise gefunden, daß die Verbindungen der allgemeinen Formel I, insbesondere die Verbindungen, für die A die 5,11-Dihydro-6H-pyrido[2,3-b][1,4]benzodiazepin-6-on-Gruppe, $R_1$ ein Wasserstoffatom und $R_2$ die Methylgruppe bedeutet, aber ganz besonders das 5,11-Dihydro-11-[1-oxo-2-methyl-6-(4-methyl-1-piperazinyl)-4-hexinyl]-6H-pyrido[2,3-b][1,4]benzodiazepin-6-on, die Enantiomeren bzw. Isomeren (vgl. hierzu DE-A-3 626 095) dieser Verbindungen sowie ihre physiologisch verträglichen Säureadditionssalze zur Therapie von Störungen der Mikrozirkulation und Behandlung peripherer Durchblutungsstörungen, insbesondere zur An wendung in der Schocktherapie, z. B. beim hämorrhagischen Schock, aber auch beim Endotoxin-Schock und traumatischen Schock, besonders geeignet sind. Die Wirksubstanz besitzt dabei nur eine äußerst geringe Affinität gegenüber muskarinischen Rezeptoren, infolgedessen treten bei therapeutischen Dosierungen keine unerwünschten Atropin-artigen Nebenwirkungen auf.

Eine kausale Therapie bei Schock und Mikrozirkulationsstörungen stand bis heute nicht zur Verfügung; bisherige Behandlungen dieser Krankheitsbilder erfolgten rein symptomatisch, z. B.
bei Schock mittels Dextran 40-Infusionen, Glucocorticoiden, Adrenalin-Bolus, Aprotinin, Dobutamin (zur Aufrechterhaltung der Nierenfuktion),
bei Mikrozirkulationsstörungen durch Gaben z. B. von Rutin, Papaverin, gelegentlich auch von $\alpha$-Blockern oder $Ca^{2+}$-Antagonisten.

Es werden zur Behandlung der obengenannten Krankheitsbilder auch nichtselektive Antimuskarinika, z. B. Atropin, N-Butylscopolammoniumbromid, Tropinbenzilat, Glykopyrroniumbromid, Oxybutynin, und myotrope Spasmolytika, z. B. Papaverin, Moxaverin, häufig auch in Kombination mit anderen Wirkstoffen, verwendet. Alle die vorstehend genannten Substanzen sind aber mit erheblichen Nebenwirkungen (z. B. am Herzen oder auf die Funktion der Speicheldrüsen) belastet. Im Gegensatz hierzu stellen die der Erfindung zugrundeliegenden Substanzen nur äußerst schwache Antimuskarinika dar, in therapeutisch relevanten

Dosen treten keine atropinartigen Nebenwirkungen auf.

Die Substanzen der allgemeinen Formel I, im besonderen das 5,11-Dihydro-11-[1-oxo-2-methyl-6-(4-methyl-1-piperazinyl)- 4-hexinyl]-6H-pyrido[2,3-b][1,4]benzodiazepin-6-on und ihre Salze bieten dagegen erstmals einen Ansatzpunkt zur kausalen Behandlung von Schockzuständen und Mikrozirkulationsstörungen.

Die Eignung von 5,11-Dihydro-11-[1-oxo-2-methyl-6-(4-methyl-1-piperazinyl)-4-hexinyl]-6H-pyrido[2,3-b]-[1,4]benzodiazepin-6-on-dihydrochlorid zur erfindungsgemäßen Anwendung bei der Schocktherapie wurde in tierexperimentellen Untersuchungen an einem Cremaster-Modell bestätigt. Dieses Modell stellt eine etablierte Methode dar, (S. Hergenröder "Mikrozirkulation im hämorragischen Schock", Diplomarbeit am Fachbereich Biologie der Joh.-Gutenberg-Universität, Mainz 1987). An diesem Modell werden Einflüsse auf den systemischen Blutdruck, die Herzfrequenz, den daraus errechneten Schockindex sowie wichtige mikrozirkulatorische Parameter, wie Arteriolendurchmesser, die Erythrozytenfließgeschwindigkeit und die daraus errechnete Mikroperfusion (Organdurchblutung) und die Überlebensrate bestimmt. Der Versuchsablauf besteht aus einer präparativen Kontrollphase, gefolgt von einer spontanen Entblutung mit einem raschen Blutdruckabfall auf 25 mmHg, einer isovolämischen intravenösen 10-minütigen Infusion mit physiologischer NaCl-Lösung (Kontrolltiere) bzw. physiologischer NaCl-Lösung mit darin gelösten Wirkstoffen und einer abschließenden Nachbeobachtungsphase (60-120 Minuten). Das zweite Modell unterscheidet sich durch eine zweitägige Nachbeobachtungsphase ohne Präparation des M. cremaster.

Das Ende der Entblutungsphase ist gekennzeichnet durch einen Stopflow im intravital beobachteten mikrozirkulären Areal, bedingt durch den drastischen Blutverlust und den damit verbundenen starken Blutdruckabfall.

Die hämodynamischen Effekte in der mit physiologischer NaCl-Lösung behandelten Kontrollgruppe waren gering und nur von kurzer Dauer mit einer Überlebensrate von nur 4 von 20 Tieren nach 60 Minuten bzw. keinem überlebenden Tier nach 120 Minuten. In der mit 5,11-Dihydro-11-[1-oxo-2-methyl-6-(4-methyl-1-piperazinyl)-4-hexinyl]-6H-pyrido[2,3-b][1,4]benzo diazepin-6-on-dihydrochlorid (2 mg/kg) behandelten Gruppe (N = 7) wurden dagegen eine Erholung der systemischen, insbesondere der mikrozirkulatorischen Parameter festgestellt. Es ergab sich eine Überlebensrate von 86 %.

Im zweiten "Schock-Modell" zeigt sich eine ähnlich hohe Überlebensrate der mit der erfindungsgemäß vorgeschlagenen Verbindung behandelten Gruppe. Während in der nicht vorbehandelten Gruppe 3 von 15 Tieren überlebten, überlebten in der mit der Wirksubstanz vorbehandelten Gruppe 13 von 15 Tieren.

Es wurden zur Untersuchung CHbb/Thom-Ratten von einem Gewicht von 100 bis 120 g herangezogen; zur Narkose diente Urethan (120 mg/100 g Ratte). Es wurden 2,0 mg/kg Wirksubstanz als Dihydrochlorid intravenös appliziert.

Als systemische Parameter wurde der Blutdruck (systolisch und diastolisch), die Herfrequenz, als mikrozirkulatorische Parameter der Gefäßdurchmesser und die Erythrozyten-Fließgeschwindigkeit gemessen und hieraus der Schockindex ( = Herzfrequenz/systolischer Blutdruck) und der Microflow (aus Gefäßdurchmesser und Erythrocyten-Fließgeschwindigkeit) berechnet.

Die hierbei gefundenen bzw. berechneten Werte sind in den Abbildungen 1 bis 7 wiedergegeben; die vorstehend genannte Wirksubstanz gemäß vorliegender Erfindung ist dort mit der Kurzbezeichnung Substanz A angegeben. Für die einzelnen Meßpunkte sind gleichzeitig die Vertrauensgrenzen angegeben.

Aus der Abbildung 1 ergibt sich für die Substanz A über den ganzen Versuchszeitraum ein sehr gleichmäßiger Kurvenverlauf des Schockindex in der Nähe der Basislinie, was für die deutliche Besserung des Schockgeschehens im Vergleich zur Kontrolle spricht. Die Abbildung 4 zeigt, daß sich der Mikroflow, d. h. die Organperfusion, durch die Substanz A ab der 60. Minute kontinuierlich erholt. Die Abbildung 7 gibt die prozentuale Überlebensrate über den Beobachtungszeitraum von 2 Stunden wieder. Es starb lediglich 1 von 7 Tieren, was einer Überlebensrate von 86 % entspricht. Von den Kontrolltieren überlebte kein Tier.

Die folgende Tabelle zeigt den prozentuellen Anteil der überlebenden Tiere bei beiden Versuchsreihen nach 2 und 48 Stunden:

Tabelle

| Prozent Überlebende | | | | |
|---|---|---|---|---|
| | 1. Versuchsserie | | 2.Versuchsserie | |
| Beobachtungsdauer | | 2 h | | 48 h |
| Kontrolle | N = 20 | 0 % | N = 10 | 20 % |
| Substanz A | N = 7 | 86 % | N = 10 | 80 % |

Aufgrund der pharmakologischen Befunde sind die Verbindungen der allgemeinen Formel I sowie ihre pharmakologisch verträglichen Säureadditionssalze zur kausalen Behandlung von Schockzuständen bzw. zur Verwendung in der Schocktherapie besonders geeignet.

Zur akuten Schockbehandlung können die Verbindungen intravenös - als Bolus-Injektion oder als Infusion - appliziert werden, zur allgemeinen Behandlung von Durchblutungsstörungen auch oral, z. B. in Form von Tabletten oder Dragées.

Der therapeutisch wirksame Dosisbereich liegt zwischen 0,02 und 10 mg/kg Körpergewicht. Die Wirksubstanzen oder ihre physiologisch verträglichen Salze lassen sich in die üblichen pharmazeutischen Zubereitungsformen, z. B. in Lösungen, Suppositorien, Tabletten, Dragées, Kapseln oder Teezubereitungen einarbeiten. Die Einzeldosis liegt im allgemeinen zwischen 0,002 und 5 mg/kg, vorzugsweise 0,05 und 1,0 mg/kg Körpergewicht, die gegebenenfalls auch in Form mehrer Einzelgaben zur Erzielung des gewünschten therapeutischen Effekts verabreicht wird.

Die Wirksubstanzen können mit Hilfe entsprechender anorganischer oder organischer Säuren auch in ihre physiologisch verträglichen Salze übergeführt werden. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Äpfelsäure als geeignet erwiesen.

Die folgenden Beispiele sollen die Herstellung einiger pharmazeutischer Anwendungsformen verdeutlichen:

<u>Beispiel I</u>

Tabletten mit 50 mg 5,11-Dihydro-11-[1-oxo-2-methyl-6-(4-methyl-1-piperazinyl)-4-hexinyl]-6H-pyrido-[2,3-b][1,4]benzodiazepin-6-on

| Zusammensetzung: | |
|---|---|
| 1 Tablette enthält: | |
| Wirkstoff | 50,0 mg |
| Milchzucker | 148,0 mg |
| Kartoffelstärke | 65,0 mg |
| Magnesiumstearat | 2,0 mg |
| | 265,0 mg |

<u>Herstellungsverfahren:</u>

Aus Kartoffelstärke wird durch Erwärmen ein l0%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 50 mg 5,11-Dihydro-11-[1-oxo-2-methyl-6-(4-methyl-1-piperazinyl)-4-hexinyl]-6H-pyrido[2,3-b][1,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 300 mg

Beispiel III

Ampullen mit 10 mg 5,11-Dihydro-11-[1-oxo-2-methyl-6-(4-methyl-1-piperazinyl)-4-hexinyl]-6H-pyrido-[2,3-b][1,4]benzodiazepin-6-on-dihydrochlorid

| Zusammensetzung: | | |
|---|---|---|
| 1 Ampulle enthält: | | |
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.

Sterilisation: 20 Minuten bei 120 °C.

Beispiel IV

Suppositorien mit 50 mg 5,11-Dihydro-11-[1-oxo-2-methyl-6-(4-methyl-1-piperazinyl)-4-hexinyl]-6H-pyrido[2,3-b][1,4]benzodiazepin-6-on

| Zusammensetzung: | |
|---|---|
| 1 Zäpfchen enthält: | |
| Wirkstoff | 50,0 mg |
| Zäpfchenmasse (z.B. Witepsol W 45$^{(R)}$) | 1 695,0 mg |
| | 1 745,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.

Zäpfchengewicht 1,745 g

Beispiel V

Tropfen mit 5,11-Dihydro-11-[1-oxo-2-methyl-6-(4-methyl-1-piperazinyl)-4-hexinyl]-6H-pyrido[2,3-b][1,4]-benzodiazepin-6-on-dihydrochlorid

| Zusammensetzung: 100 ml Tropflösung enthalten: | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 5,0 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Ansprüche**

1.) Verwendung von Diazepinonen der allgemeinen Formel I

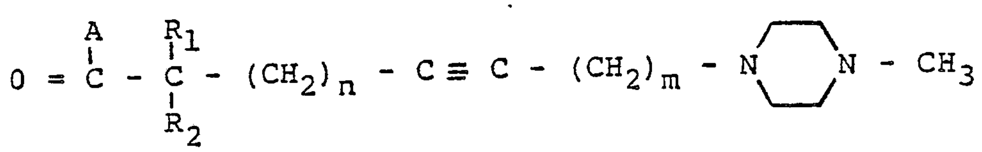
, (I)

in der
A die in 11-Stellung gebundene 5,11-Dihydro-6H-pyrido[2,3-b][1,4]benzodiazepin-6-on-, die in 5-Stellung gebundene 5,10-Dihydro-11H-dibenzo[b,e][1,4]diazepin-11-on-, die in 5-Stellung gebundene 5,11-Dihydro-10H-pyrido[3,2-b][1,4]benzodiazepin-10-on- oder die in 4-Stellung gebundene 4,9-Dihydro-10H-thieno[3,4-b][1,5]benzodiazepin-10-on-Gruppe, die in 1- und/oder in 3-Stellung durch Alkylreste mit 1 bis 4 Kohlenstoffatomen oder in 3-Stellung durch Chlor, Fluor oder Brom substituiert sein kann, die in 11-Stellung gebundene 6,11-Dihydro-5H-pyrido[2,3-b][1,5]benzodiazepin-5-on- oder die in 4-Stellung gebundene 1-Methyl-1,4,9,10-tetrahydropyrrolo[3,2-b][1,5]benzodiazepin-10-on-Gruppe, die in 3-Stellung durch Methyl oder Chlor substituiert sein kann,
$R_1$ ein Wasserstoffatom oder eine Alkylgruppe mit 1 bis 3 Kohlenstoffatomen,
$R_2$ eine Alkylgruppe mit 1 bis 3 Kohlenstoffatomen, und
n und m unabhängig voneinander die ganzen Zahlen 1 bis 3 bedeuten, gegebenenfalls ihrer Enantiomeren bzw. Isomeren und ihrer physiologisch verträglichen Säureadditionssalze zur Therapie von Störungen der Mikrozirkulation bzw. der pheripheren Durchblutung und in der Schocktherapie.

2.) Verwendung von Diazepinonen der allgemeinen Formel I

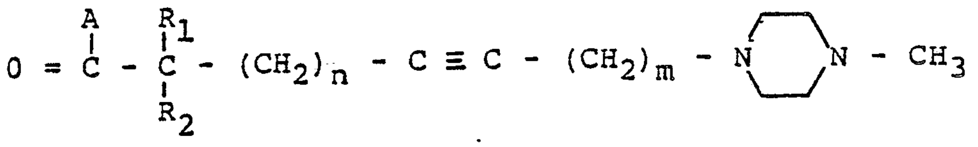

, (I)

in der

A die in 11-Stellung gebundene 5,11-Dihydro-6H-pyrido[2,3-b][1,4]benzodiazepin-6-on-, die in 5-Stellung gebundene 5,10-Dihydro-11H-dibenzo[b,e][1,4]diazepin-11-on-, die in 5-Stellung gebundene 5,11-Dihydro-10H-pyrido[3,2-b][1,4]benzodiazepin-10-on- oder die in 4-Stellung gebundene 4,9-Dihydro-10H-thieno[3,4-b][1,5]benzodiazepin-10-on-Gruppe, die in 1- und/oder in 3-Stellung durch Alkylreste mit 1 bis 4 Kohlenstoffatomen oder in 3-Stellung durch Chlor, Fluor oder Brom substituiert sein kann, die in 11-Stellung gebundene 6,11-Dihydro-5H-pyrido[2,3-b][1,5]benzodiazepin-5-on- oder die in 4-Stellung gebundene 1-Methyl-1,4,9,10-tetrahydropyrrolo[3,2-b][1,5]benzodiazepin-10-on-Gruppe, die in 3-Stellung durch Methyl oder Chlor substituiert sein kann,

$R_1$ ein Wasserstoffatom oder eine Alkylgruppe mit 1 bis 3 Kohlenstoffatomen,

$R_2$ eine Alkylgruppe mit 1 bis 3 Kohlenstoffatomen, und

n und m unabhängig voneinander die ganzen Zahlen 1 bis 3 bedeuten, gegebenenfalls ihrer Enantiomeren bzw. Isomeren und ihrer physiologisch verträglichen Säureadditionssalze zur Herstellung eines Arzneimittels zur Therapie von Störungen der Mikrozirkulation bzw. der peripheren Durchblutung und für die Schocktherapie.

3.) Verwendung von 5,11-Dihydro-11-[1-oxo-2-methyl-6-(4-methyl-1-piperazinyl)-4-hexinyl]-6H-pyrido-[2,3-b] [1,4]-benzodiazepin-6-on und seiner physiologisch verträglichen Säureadditionssalze zur Therapie von Störungen der Mikrozirkulation bzw. der peripheren Durchblutung.

4.) Verwendung von 5,11-Dihydro-11-[1-oxo-2-methyl-6-(4-methyl-1-piperazinyl)-4-hexinyl]-6H-pyrido-[2,3-b][1,4]benzodiazepin-6-on und seiner physiologisch verträglichen Säureadditionssalze zur Anwendung in der Schocktherapie.

5.) Verwendung von 5,11-Dihydro-11-[1-oxo-2-methyl-6-(4-methyl-1-piperazinyl)-4-hexinyl]-6H-pyrido-[2,3-b][1,4]benzodiazepin-6-on und seiner physiologisch verträglichen Säureadditionssalze zur Herstellung eines Arzneimittels zur Therapie von Störungen der Mikrozirkulation bzw. der peripheren Durchblutung.

6.) Verwendung von 5,11-Dihydro-11-[1-oxo-2-methyl-6-(4-methyl-1-piperazinyl)-4-hexinyl]-6H-pyrido-[2,3-b][1,4]benzodiazepin-6-on und seiner physiologisch verträglichen Säureadditionssalze zur Herstellung eines Arzneimittels zur Anwendung in der Schocktherapie.

ABB.1.

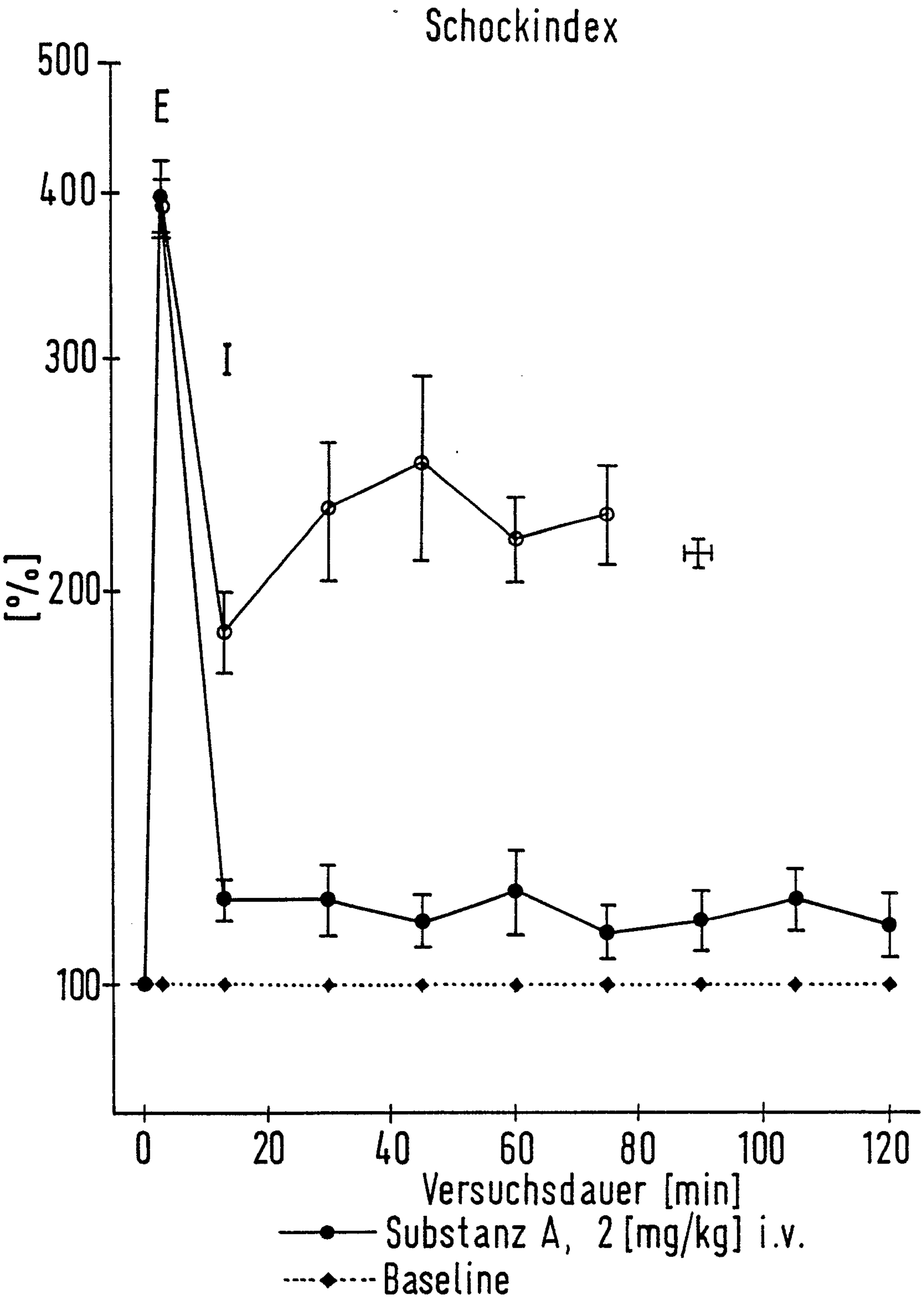
Schockindex
500
400
300
200
100
[%]
E
0
20
40
60
80
100
120
Versuchsdauer [min]
Substanz A, 2 [mg/kg] i.v.
Baseline
NaCl-Kontrolle

ABB.2.

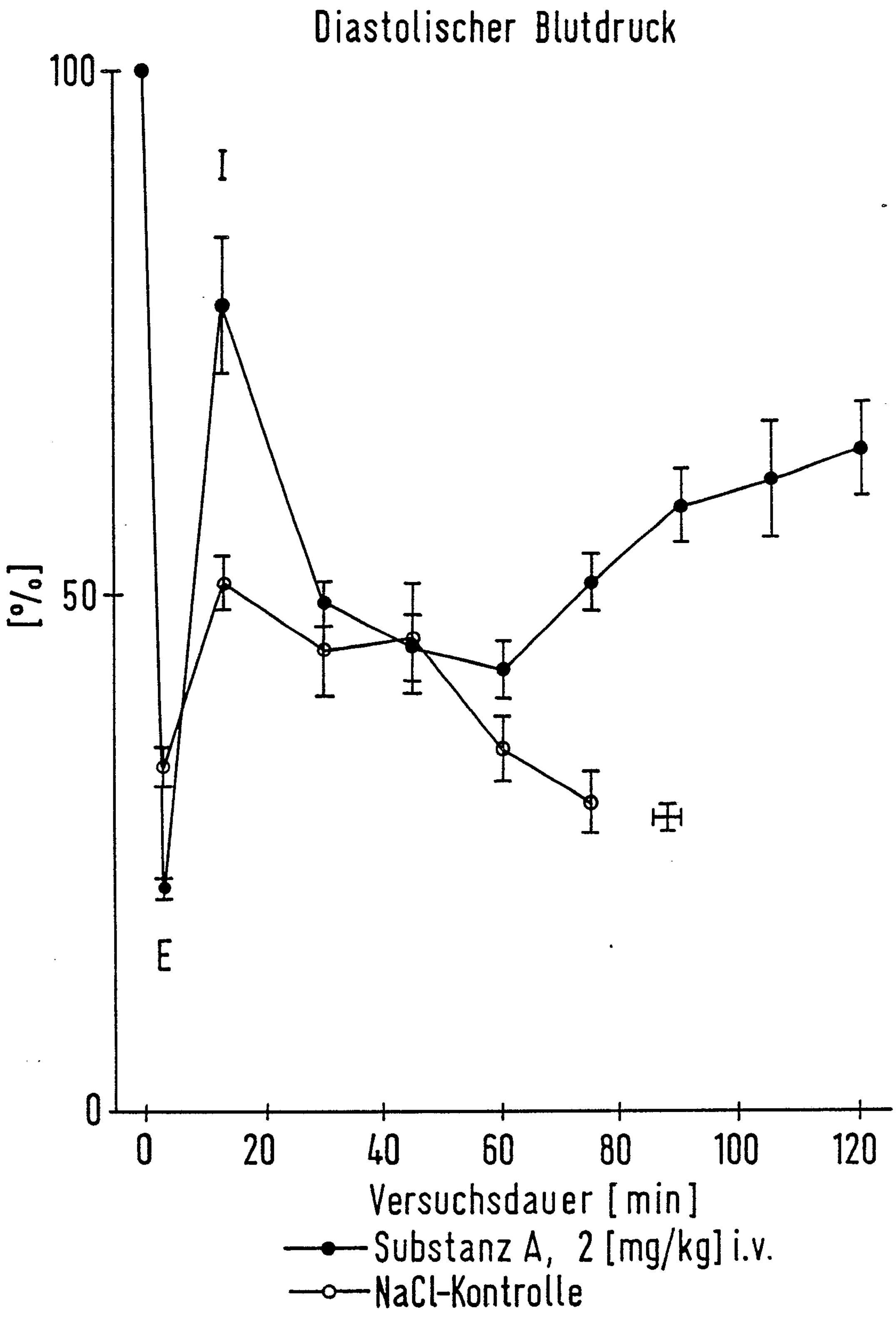
Diastolischer Blutdruck
100
50
0
[%]
I
E
0
20
40
60
80
100
120
Versuchsdauer [min]
Substanz A, 2 [mg/kg] i.v.
NaCl-Kontrolle

ABB.3.

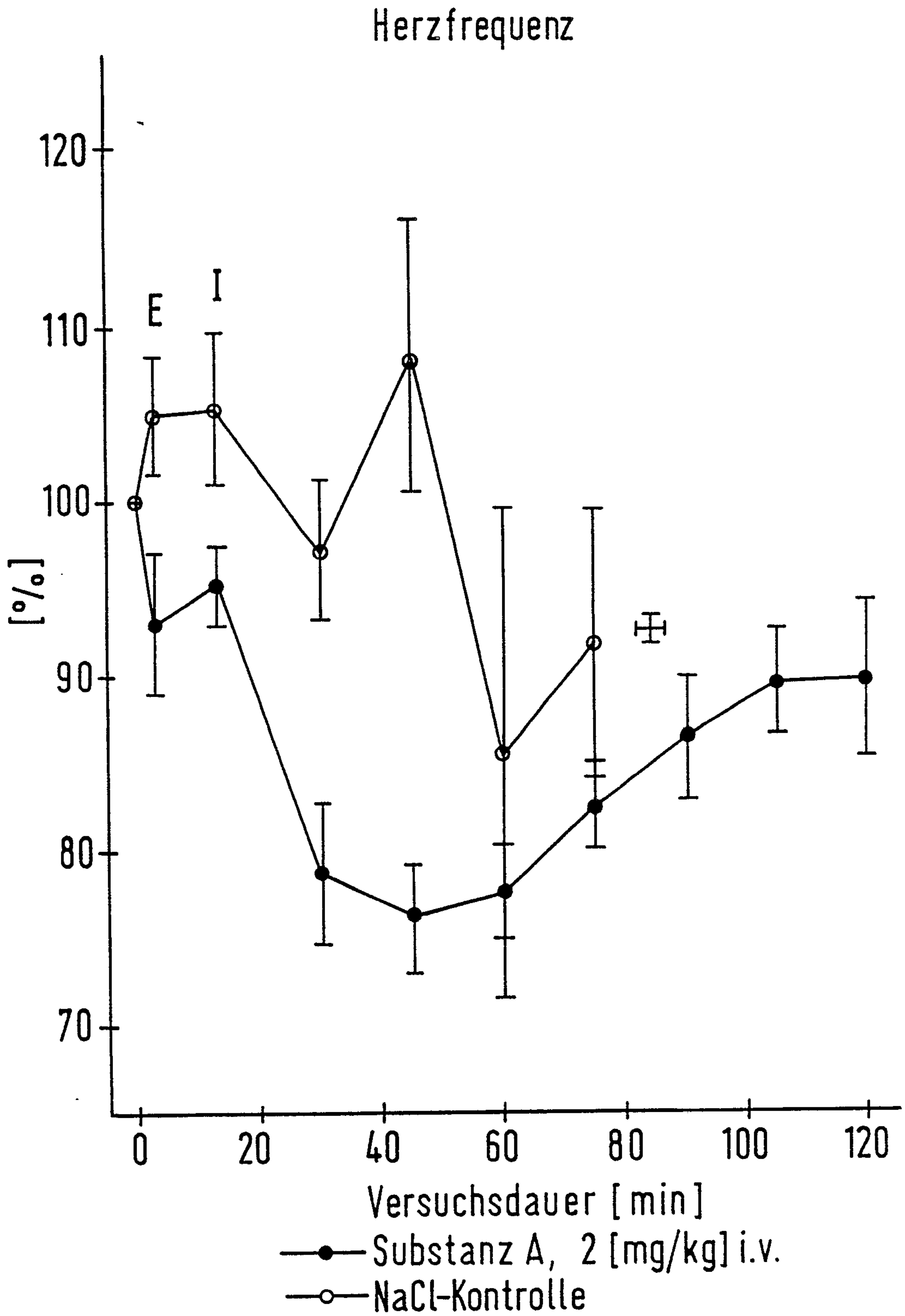
Herzfrequenz
120
110
100
90
80
70
[%]
E
I
0
20
40
60
80
100
120
Versuchsdauer [min]
Substanz A, 2 [mg/kg] i.v.
NaCl-Kontrolle

ABB.4.

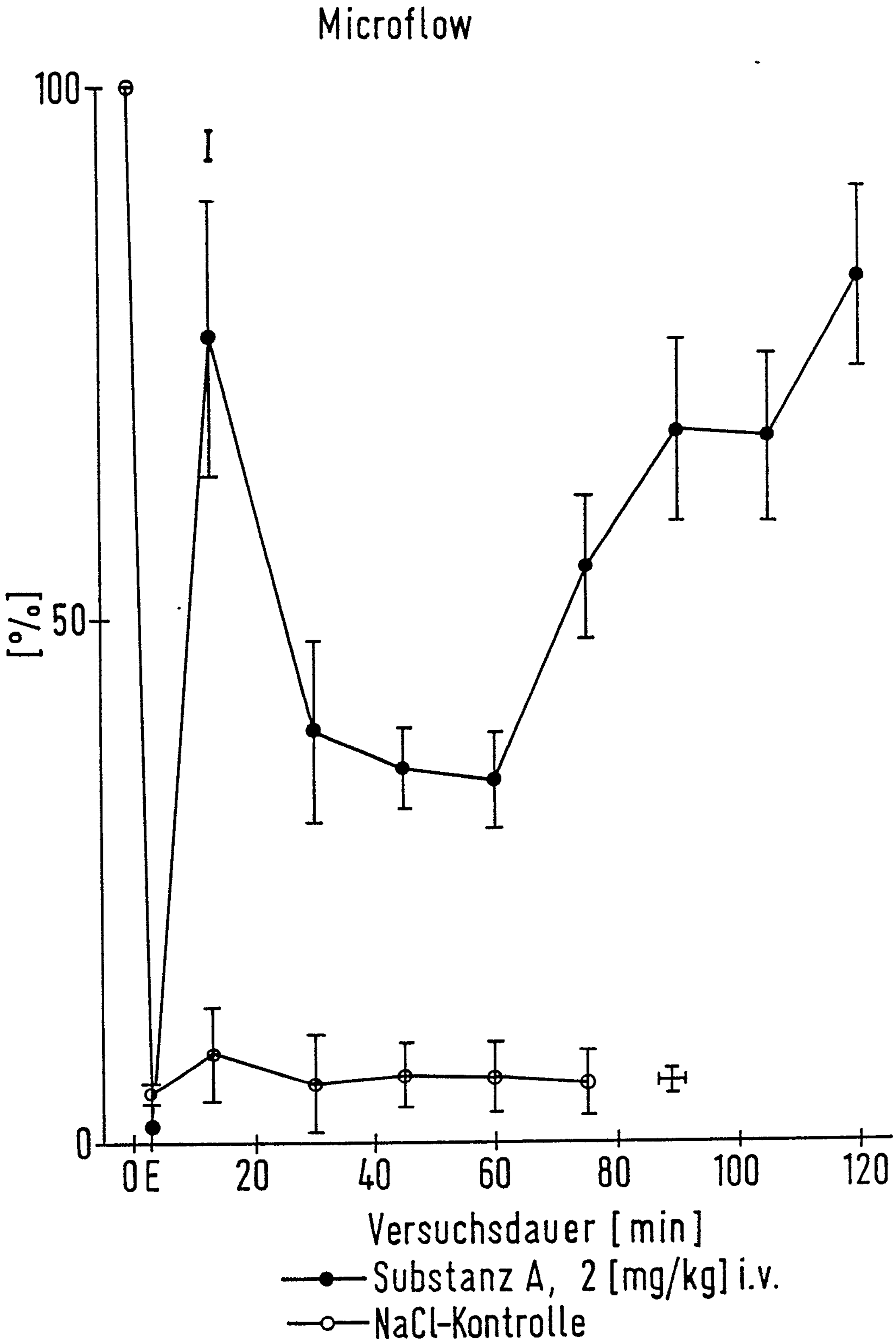
Microflow
100
50
0
[%]
0 E
20
40
60
80
100
120
Versuchsdauer [min]
Substanz A, 2 [mg/kg] i.v.
NaCl-Kontrolle

ABB.5.

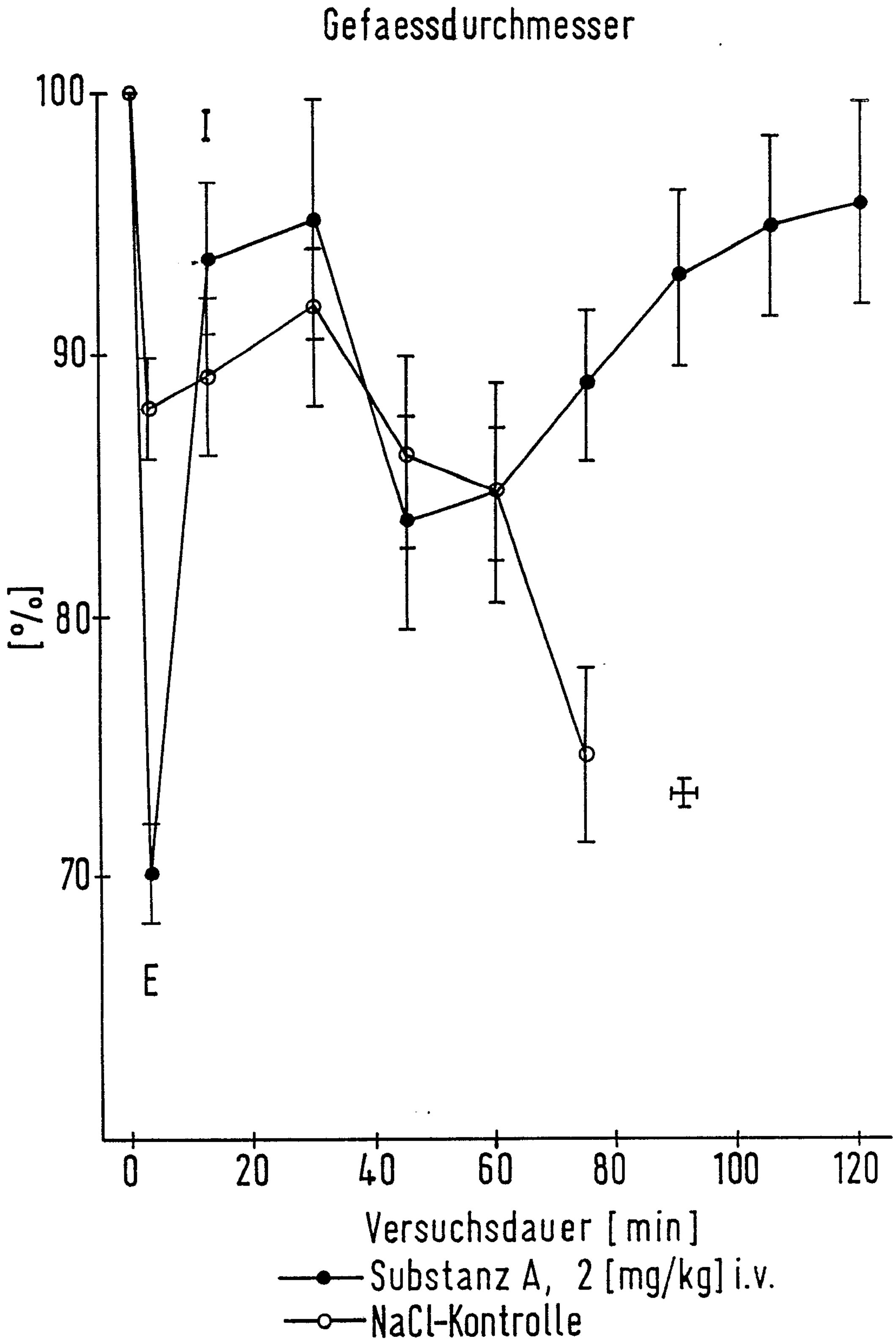
Gefaessdurchmesser
100
90
80
70
[%]
E
0
20
40
60
80
100
120
Versuchsdauer [min]
Substanz A, 2 [mg/kg] i.v.
NaCl-Kontrolle

# ABB.6.

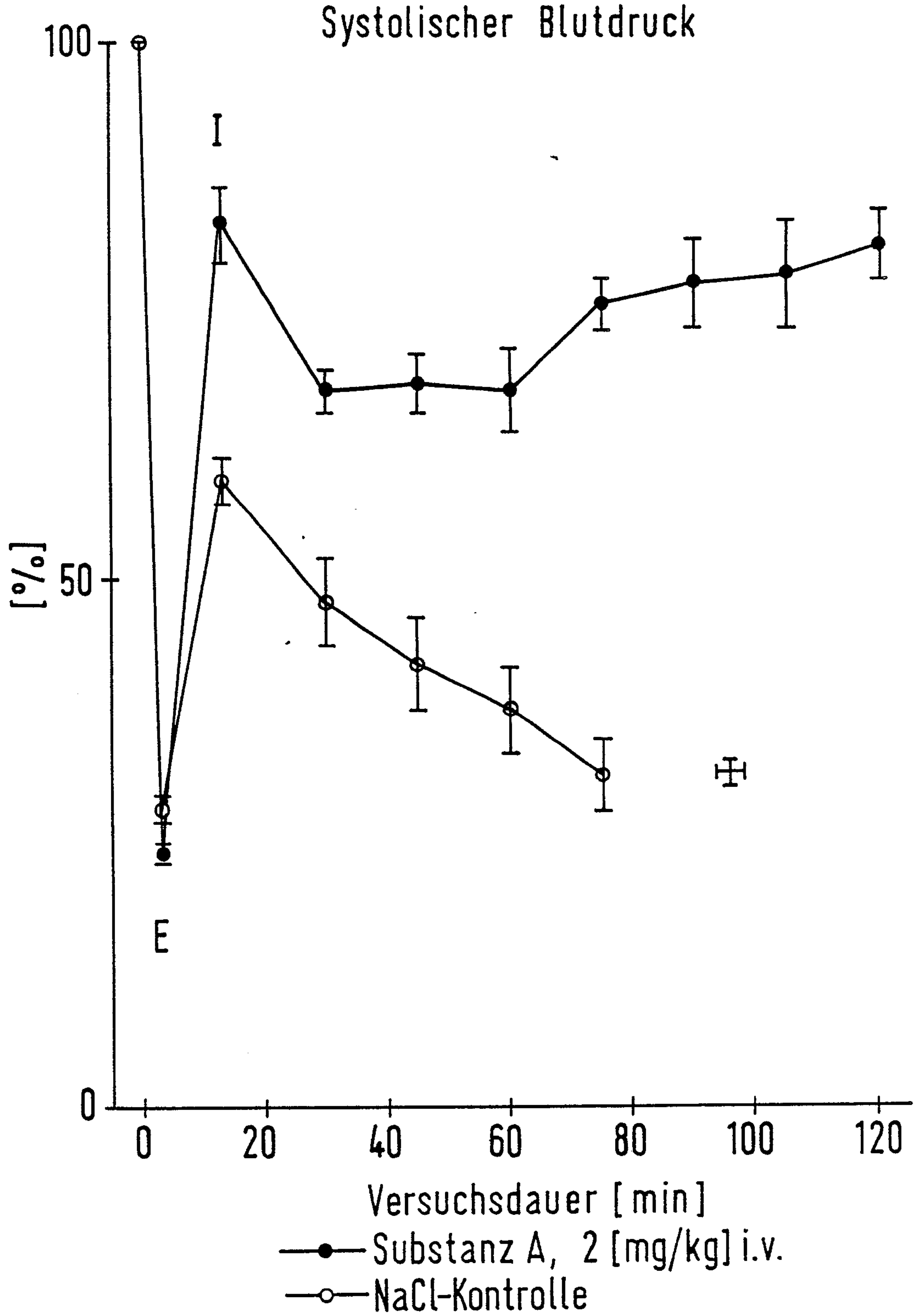
Systolischer Blutdruck
100
50
0
[%]
I
E
0
20
40
60
80
100
120
Versuchsdauer [min]
Substanz A, 2 [mg/kg] i.v.
NaCl-Kontrolle

ABB.7.

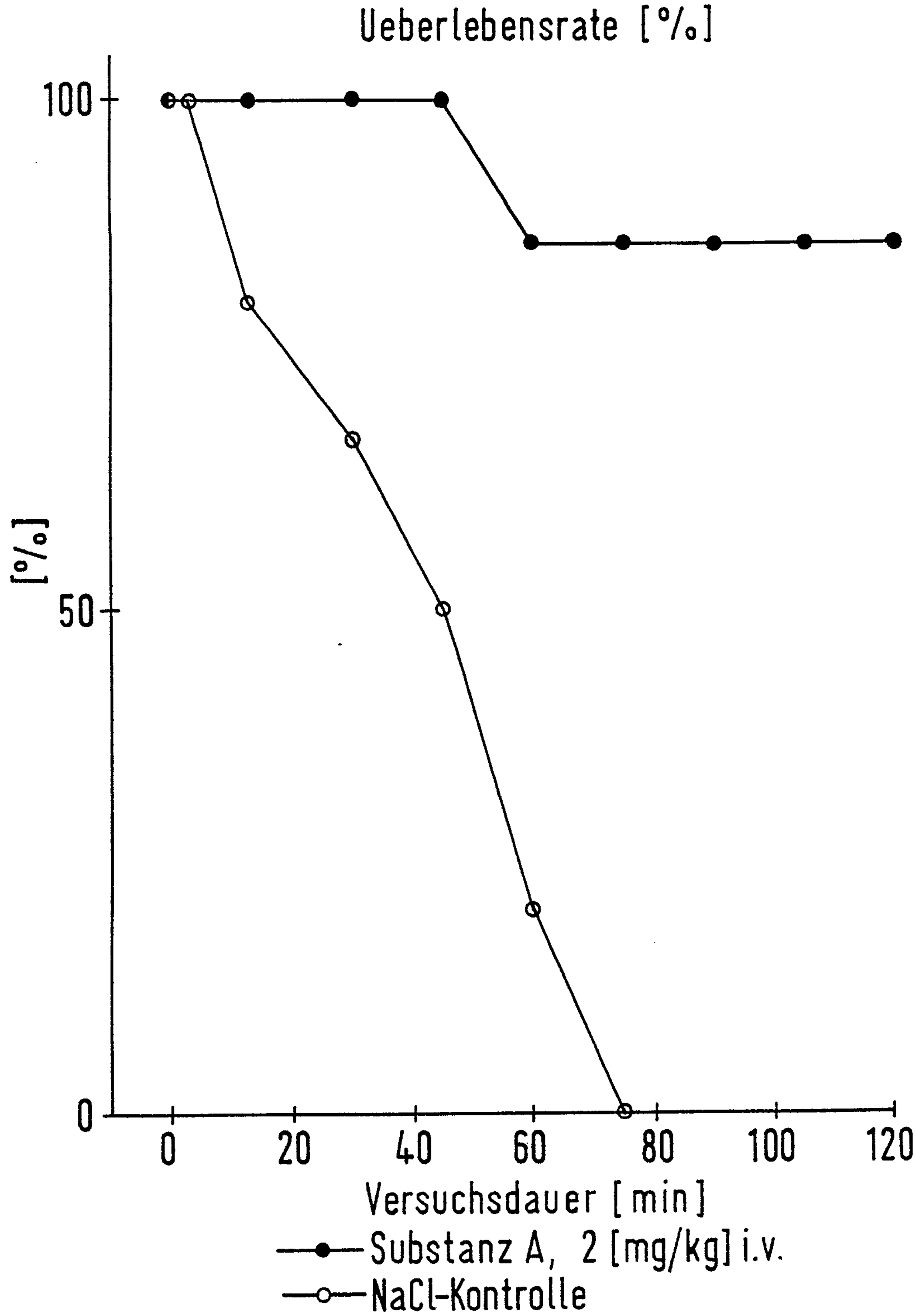
Ueberlebensrate [%]
100
50
0
[%]
0
20
40
60
80
100
120
Versuchsdauer [min]
Substanz A, 2 [mg/kg] i.v.
NaCl-Kontrolle